# FASCICULE DE BREVET EUROPEEN

(11) **EP 3 498 220 B1**
(45) Date de publication et mention de la délivrance du brevet: **25.03.2020**
(21) Numéro de dépôt: 18000966.4
(22) Date de dépôt: 13.12.2018
(51) Int. Cl.: A61C 8/00

(54) **IMPLANT DENTAIRE**
ZAHNIMPLANTAT
DENTAL IMPLANT

(30) Priorité: 13.12.2017 BE 201700171
(43) Date de publication de la demande: 19.06.2019
(73) Titulaire: Sudimplant, 31500 Toulouse (FR)
(72) Inventeur: Benhamou, Olivier, 1060 Bruxelles (BE)
(74) Mandataire: Quintelier, Claude

(56) Documents cités:
- EP-A1- 1 418 858
- EP-A1- 2 586 398
- EP-A1- 2 845 562
- CH-A2- 702 586
- JP-A- H0 449 958

## Description

La présente invention concerne un implant dentaire comprenant un noyau fabriqué en métal et un manchon ayant une paroi intérieure et monté par emboîtement sur une partie supérieure du noyau de telle façon que la paroi intérieure est située face à cette partie supérieure du noyau, lequel manchon est fabriqué en céramique ou en Peek, un écart étant laissé entre le noyau et la paroi intérieure du manchon de telle façon à ce qu'une couche de colle puisse être logée dans un espace formé par la présence de l'écart.

Un tel implant dentaire est connu du brevet EP 1 418 858. Dans l'implant dentaire connu le manchon est collé sur le noyau. A cette fin un écart situé entre 80 et 150 µm est laissé entre la paroi intérieure du manchon et la partie supérieure du noyau. Cet écart permet de former un espace suffisant pour permettre une bonne élasticité entre le manchon et le noyau.

Un désavantage de l'implant connu est qu'il y a un risque de décollement. En effet, il a été constaté que lors ou après l'insertion de ces implants dans les os maxillaires denses ou irréguliers, ces derniers peuvent parfois repousser le manchon et ainsi provoquer que le collage lâche, causant ainsi que le manchon se détache du noyau. Ces implants n'étaient dès lors pas indiqués pour être enfouis totalement dans l'os des maxillaires, et le manchon ne pouvait pas être en contact de l'os.

L'invention a pour bût de réaliser un implant dentaire où le risque de décollement et de fracture est considérablement réduit.

A cette fin un implant suivant l'invention est caractérisé en ce que ledit écart a une distance située entre 5 et 30 µm, en particulier entre 10 et 20 µm. Cette valeur de l'écart permet de façon inattendue de combiner à la fois une bonne résistance à l'insertion et au contact de l'os d'une part et d'autre part de garder suffisamment d'espace entre le noyau et le manchon pour permettre une certaine élasticité entre le manchon et le noyau. Cette valeur de l'écart permet aussi un meilleur résultat au test noyau. Cette valeur de l'écart permet aussi un meilleur résultat au test d'arrachement du manchon fixé sur le noyau et réduit la probabilité de fracture lors de ce test. Ce manchon peut alors être partiellement ou intégralement enfoui dans l'os, et cet implant peut également être totalement enfoui dans l'os des maxillaires, même irrégulier,

Une première forme de réalisation préférentielle d'un implant dentaire suivant l'invention est caractérisée en ce que la céramique dont est fabriqué le manchon est de la zircone. La zircone est une matière qui possède d'excellentes propriétés de biocompatibilité, en particulier au contact des tissus mous.

Une deuxième forme de réalisation préférentielle d'un implant dentaire suivant l'invention est caractérisée en ce que la couche de colle est une couche de colle époxy bi-composant biocompatible. Cette colle est bien appropriée pour un usage implantable dentaire.

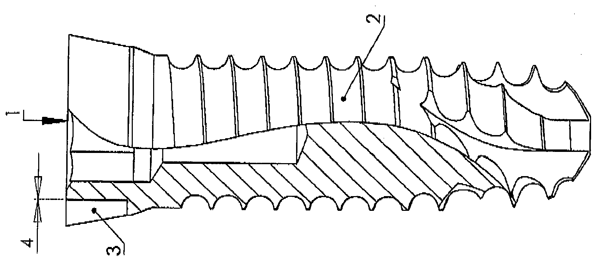

L'invention sera maintenant décrite plus en détails à l'aide de l'unique dessin joint en annexe et qui décrit une forme de réalisation d'un implant dentaire selon l'invention.

Le dessin illustre une vue en coupe d'un implant dentaire 1 suivant l'invention. L'implant dentaire comporte un noyau 2 fabriqué en métal, en particulier du titane. Le titane a l'avantage d'être résistant, flexible et biocompatible pour un usage d'implants dentaire dans la bouche d'un patient. Le noyau possède de préférence un pas de vis externe permettant de visser le noyau dans l'os des maxillaires.

Un manchon 3 est placé sur une partie supérieure du noyau et s'étend sur tout le pourtour du noyau. Le manchon possède une paroi intérieure et est monté par emboîtement sur une partie supérieure du noyau, de telle façon que la paroi intérieure est située face à cette partie supérieure du noyau lorsque l'implant est implanté dans l'arcade maxillaire ou mandibulaire du patient, le manchon se positionne dans la gencive, entre l'os et la cavité buccale.

Le manchon est de préférence fabriqué en céramique, plus particulièrement en zircone. Le manchon peut également être fabriqué en Peek.

Un écart 4 est laissé entres le noyau et la paroi intérieure du manchon de telle façon à ce qu'une couche de colle puisse être logée dans un espace formé par la présence de l'écart. L'écart a une distance située entre 5 et 30 µm, en particulier entre 10 et 20 µm. Cet écart s'étend sur toute la hauteur du manchon de telle façon à d'une part faciliter l'emboîtement du manchon sur la partie supérieure du noyau, et d'autre part de permettre à la couche de colle de s'étendre sur toute cette hauteur et ainsi permettre une bonne adhérence et un bon assemblage entre le manchon et le noyau.

La colle utilisée pour coller le manchon au noyau est de préférence une colle époxy bi-composant. Cette colle est bien tolérée pour un usage buccal d'implants dentaire, car elle est biocompatible.

L'avantage de laisser un écart ayant une distance située entre 5 et 30 µm, en particulier entre 10 et 20 µm, est que cela permet de façon inattendue de combiner à la fois une bonne résistance de ce manchon au contact de l'os des maxillaires d'une part, et d'autre part de garder suffisamment d'espace entre le noyau et le manchon pour permettre une certaine élasticité entre le manchon et le noyau en raison des forces masticatoires appliquées. En effet, un problème de détachement du manchon du noyau a été constaté sur les implants dentaire connus de l'art antérieur, Une analyse approfondie de ce problème a permis de constater que l'os des maxillaires peut parfois repousser le manchon de l'implant lorsqu'il est implanté dans la bouche du patient. Ceci est notamment dû au fait que l'os n'est pratiquement jamais parfaitement plat et que même de petites irrégularités de la crête osseuse, de l'ordre de moins d'un millimètre, provoquent déjà une pression sur le manchon. Ainsi cette pression exercée par l'os des maxillaires sur le manchon peut provoquer que le collage et l'assemblage lâchent lors de la pose de l'implant, causant ainsi que le manchon se détache du noyau.

Cette valeur de l'écart ayant une distance située entre 5 et 30 µm, en particulier entre 10 et 20 µm, permet aussi un meilleur résultat au test d'arrachement du manchon fixé sur le noyau et réduit la probabilité de fracture lors de ce test. Ces tests sont effectués en usine lors de la fabrication de l'implant afin de vérifier la résistance à l'arrachement du manchon fixé sur le noyau. Ces tests ont permis de constater que même avec une aussi fine couche de colle on obtenait suffisamment d'élasticité entre le manchon et le noyau pour éviter une fracture du manchon.

Un autre avantage de cet écart selon l'invention est que le risque d'infiltration de bactéries dans l'espace laissé par l'écart est minimal. En effet eu égard à cette distance très petite de l'écart et dû au fait que l'espace formé par l'écart est rempli de cette colle biocompatible, il ne reste pour ainsi dire plus de place pour les bactéries.

## Revendications

1. Implant dentaire comprenant un noyau fabriqué en métal et un manchon ayant une paroi intérieure et monté par emboîtement sur une partie supérieure du noyau de telle façon que la paroi intérieure est située face à cette partie supérieure du noyau, lequel manchon est fabriqué en céramique ou en Peek, un écart étant laissé entre le noyau et la paroi intérieure du manchon de telle façon à ce qu'une couche de colle puisse être logée dans un espace formé par la présence de l'écart, **caractérisé en ce que** ledit écart a une distance située entre 5 et 30 µm, en particulier entre 10 et 20 µm.

2. Implant dentaire suivant la revendication 1, **caractérisé en ce que** la céramique dont est fabriqué le manchon est de la zircone.

3. Implant dentaire suivant la revendication 1 ou 2, **caractérisé en ce que** la couche de colle est une couche de colle époxy bi-composant biocompatible.

## Patentansprüche

1. Zahnimplantat welches ein aus Metall hergestellten Kern enthält und eine Manschette mit einem inneren Wand und durch Einhüllung auf ein oberen Teil des Kernes derart montiert dass den inneren Wand sich gegenüber dieses oberen Teil des Kernes befindet, welcher Manschette aus Keramik oder Peek hergestellt ist, eine Lücke zwischen den Kern und den inneren Wand der Manschette ist derart gebildet dass eine Klebestoffschicht in einen Raum gebildet durch die Anwesenheit dieser Lücke untergebracht werden kann, **dadurch gekennzeichnet dass** genannte Lücke eine Distanz zwischen 5 und 30 µm, ins besondere zwischen 10 und 20 µm, aufweist.

2. Zahnimplantat nach Anspruch 1, **dadurch gekennzeichnet, dass** der Keramik, aus welcher die Manschette gebildet ist Zirconium ist.

3. Zahnimplantat nach Anspruch 1 oder 2, **dadurch gekennzeichnet, dass** die Klebestoffschicht eine bi-Komponente biokompatiblen epoxy Klebeschicht ist.

## Claims

1. A dental implant comprising a core manufactured in metal and a sleeve having an internal wall and nested by sliding on an upper part of the core in such a manner that the internal wall is located faced to that upper part of the core, which sleeve is manufactured from ceramic or Peek, a distance being left between the core and the internal wall of the sleeve in such a manner that an adhesive layer can be lodged in a space formed by the presence of that distance, **characterised in that** said space has a distance situated between 5 and 30 µm, in particular between 10 and 20µm.

2. The dental implant as claimed in claimed in claim 1, **characterised in that** the ceramic of which the sleeve is manufactured is zirconium.

3. The dental implant as claimed in claimed in claim 1 or 2, **characterised in that** the adhesive layer is a biocompatible bi-component adhesive layer.
